(19) Europäisches Patentamt
European Patent Office
Office européen des brevets

(11) EP 4 721 667 A1

(12) EUROPEAN PATENT APPLICATION

(43) Date of publication:
08.04.2026 Bulletin 2026/15

(21) Application number: 24204407.1

(22) Date of filing: 03.10.2024

(51) International Patent Classification (IPC):
*A61B 6/00* (2024.01)  *G06T 11/00* (2026.01)
*A61B 6/03* (2006.01)

(52) Cooperative Patent Classification (CPC):
**A61B 6/5205; G06T 12/10;** A61B 6/032

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **DAERR, Heiner**
**Eindhoven (NL)**
• **SCHAEFER, Dirk**
**Eindhoven (NL)**

(74) Representative: **Philips Intellectual Property & Standards**
**High Tech Campus 52**
**5656 AG Eindhoven (NL)**

(54) **FAST READOUT BINNING FOR X-RAY SYSTEMS**

(57) System (FCS) and related method for X-ray imaging. The system comprises an input interface (IN) through which is receivable detector data detectable by an X-ray detector (XD) of an X-ray imaging apparatus (IA) upon irradiation by X-radiation from a focal spot (FS) of the imaging apparatus (IA)'s X-ray source (XR). A binner (BN) bins the detector data into binned detector data. The said binning by binner (BN) is based on a manner in which a spatial extent of a projection on the detector (XD) of the focal spot (FS) is changeable along at least one spatial direction $X,Z$.

Fig. 6

**Description**

FIELD OF THE INVENTION

The invention relates to a system for X-ray imaging, to an imaging arrangement including such as system, to a related method, a computer program element, and a computer-readable medium.

BACKGROUND OF THE INVENTION

Imaging, in particular x-ray imaging, is one of the most important tools in the arsenal of the modem medic. Imaging allows obtaining, in a non-invasive manner, information about internal anatomical structure and constitution of a patient which can quickly inform diagnosis or treatment. In an ever-aging population, particular in the higher income world, a demand for imaging services has soared. Combined with this, is the ever more sophisticated imaging equipment itself which allows acquiring multi-channel and/or high resolution imagery, which leaves vast amounts of data to be processed.

Whilst emergence of such high resolution data as such is welcome as it allows yet better diagnosis and gathering of intelligence, it does come at a resource cost, with unique set of challenges: such vast amounts of data need to be stored, processed and transmitted. Read-out circuitry in particular in x-ray detectors have been found to form a bottleneck. Reading out data from pixel at ever smaller size and thus from detectors with associated high pixel density, is time consuming. Binning has been proposed as a solution. However, this too comes at a cost, as information may be lost.

SUMMARY OF THE INVENTION

Thus, what is needed is a system operable to secure fast read-outs, whilst capable of balancing this usefully versus image resolution loss.

An object of the present invention is achieved by the subject matter of the independent claims where further embodiments are incorporated in the dependent claims. It should be noted that the following described aspect of the invention equally applies to the related methods, to the imaging arrangement, to the computer program element and to the computer readable medium.

According to a first aspect of the invention there is provided a system for X-ray imaging, comprising :-

- input interface through which is receivable detector data detectable by an X-ray detector of an X-ray imaging apparatus upon irradiation by X-radiation from a focal spot of the imaging apparatus 's X-ray source; and
- a binner capable of binning (combining) the detector data into binned (combined) detector data, and wherein the said binning by binner is based on a manner in which a spatial extent of a

projection on the detector of the focal spot is changeable along at least one spatial direction in an examination region of the imaging apparatus.

Specifically, the binning by binner is based on the manner in in which the spatial extent of the projection (footprint) on the detector of the focal spot is changeable with position on the detector's imaging surface (L) along the said at least one spatial direction.

In embodiments, the said change in spatial extent is a result of perspective distortion.

In embodiments, the binning is based on a 2D (2-dimensional) binning kernel, wherein a spatial extent of the binning kernel is variable with, and corresponds to, the said change of spatial extent of the projection of the focal spot.

In embodiments, the spatial extent of the binning kernel is the larger, the larger the spatial extent of the projection of the focal spot.

In embodiments, the varying/variability is to make spatial extent of the binning kernel correspond to the said projective spatial extent.

In embodiments, the said binning includes fractional binning from pixels. The fractional binning may include interpolation of pre-read-out data from pixels.

In embodiments, the imaging apparatus is of the radiography type, or of the tomographic type.

In embodiments, the apparatus is any one of: a computed tomography, CT, scanner, a cone beam scanner, CB(cone-beam)CT, a C-arm scanner, a radiography apparatus.

In embodiments, the said direction is any one or more of: i) direction of a rotation axis (Z) of the x-ray source and/or of the X-ray detector, and ii) a direction (X) in a plane perpendicular to said, or a rotation axis. The direction may be one in an examination region of the imaging apparatus, and may be conceptualized as projected onto the detector's imaging surface. The direction is preferably one that is not parallel to a normal of the detector's imaging surface. The rotation may be virtual such as in some higher generation CT scanner systems.

In embodiments, at least a part (such as the binner) of the system is integrable in the X-ray detector.

In embodiments, the system is integrable in detector readout circuitry.

In embodiments, the binning is based on operational data, wherein the calibration data is based on prior recorded X-ray measurements indicative of spatial extent of projection of focal spot.

In another aspect there is provided an imaging arrangement including the said system and any one or more of: a readout-electronics of the X-ray detector, the X-ray detector, the imaging apparatus, and a memory including the calibration/operational data.

In another aspect there is provided a method of X-ray imaging, comprising:

- receiving detector data detectable by an X-ray detector of an X-ray imaging apparatus upon irradiation by X-radiation from a focal spot of the imaging apparatus's X-ray source; and

- binning the detector data into binned detector data, and wherein the said binning by binner is based on a manner in which a spatial extent of a projection on the detector of the focal spot is changeable along at least one spatial direction in an examination region of the imaging apparatus.

In another aspect there is provided a method of obtaining calibration data for use in the method of X-ray imaging, wherein the binning is based on such calibration data.

In another aspect there is provided a computer program element, which, when being executed by at least one processing unit, is adapted to cause the processing unit to perform the method of X-ray imaging or of the method of obtaining calibration data.

In another aspect there is provided at least one computer readable medium having stored thereon the program element.

Thus, in other words, as proposed herein, the binning applied or applicable at a particular location on the detector's imaging surface is based on, in particular is dependent on, a perspective distortion under projection of (a face of) the focal spot as viewed from the said respective particular location.

In more detail, what is proposed herein is to apply spatially dependent binning taking the focal spot blurring into account. The system and method are configured to strike a in particular clinically useful balance between readout time and the final spatial resolution of projection imagery. The perspective distortion of spatial extent (size and/or shape) of focal spot of the X-ray source as may be seen by a given detector pixel depends on the location of the pixel on the X-ray detector. For example, the focal spot face size increases along, say, the Z-axis (rotation axis) of a tomographic imager. On the other hand, in CT, the focal spot size along the x-direction (perpendicular to axis Z) remains approximal the same, although the focal spot projection footprint as seen at the respective detector pixel region experiences apparent skewing/perspective distortion along the X-axis, which does increase the effective (detector recordable) focal spot projection footprint size. The effective focal spot projection footprint size can be used herein to improve the binning scheme for different detector shapes/layouts of different X-ray imaging systems, such as CT, CBCT, C-arm, etc.

Thus, with the proposed binning system, higher resolution is used at locations where it is due, whilst at others it is not, thus leading to a net saving in processing, and in particular read-out time over conventional approaches. Such conventional binning, approaches whilst possibly fast, may forsake valuable resolution, potentially obscuring crucial intelligence in the obtained imagery. Conversely, whilst other approaches attempt to maintain high resolution, such approaches may suffer from slower imaging. The above proposed binning strikes a useful balance resolution and speed and may be used in particular with benefit in fast imaging settings.

Whilst in the following main reference will be made to tomographic imaging as said purely projection-based imaging where no reconstruction is done such as in radiography and may be used in trauma centers, orthopedic centers, or others are not excluded herein

BRIEF DESCRIPTION OF THE DRAWINGS

Exemplary embodiments of the invention will now be described with reference to the following drawings, which, unless stated otherwise, are not to scale, wherein:

Fig. 1 shows a block diagram of an x-ray imaging arrangement;
Fig. 2 shows operation of an x-ray source;
Fig. 3 is a side view of an x-ray beam issuing forth from a focal spot of an x-ray source for detection at X-ray detector's image plane/surface;
Fig. 4 affords projection view of focal spot projection footprint as a function of position along various lines through the examination region/along the detector plane/surface;
Fig. 5 shows the operation of an x-ray detector in more detail, in particular of its read-out circuitry;
Fig. 6 shows a block diagram of operation of a proposed binning system as envisaged herein in some embodiments;
Fig. 7 illustrates variation of focal spot size with distance along the rotation axis of a tomographic imaging apparatus;
Fig. 8 shows a flow chart of a computing-implemented method of facilitating x-ray imaging, in particular for improved balancing or tradeoff of read-out speed vs resolution;
Fig. 9 shows calibration data as may be used in obtaining data for use by the proposed binning system;
Fig. 10 shows curves of resolution versus off-center distance in a reconstructed CT image; and
Fig. 11 is another graph that illustrates the proposed binning scheme in heavy lines, again as resolution versus off-center distance as in Fig 10.

DETAILED DESCRIPTION OF EMBODIMENTS

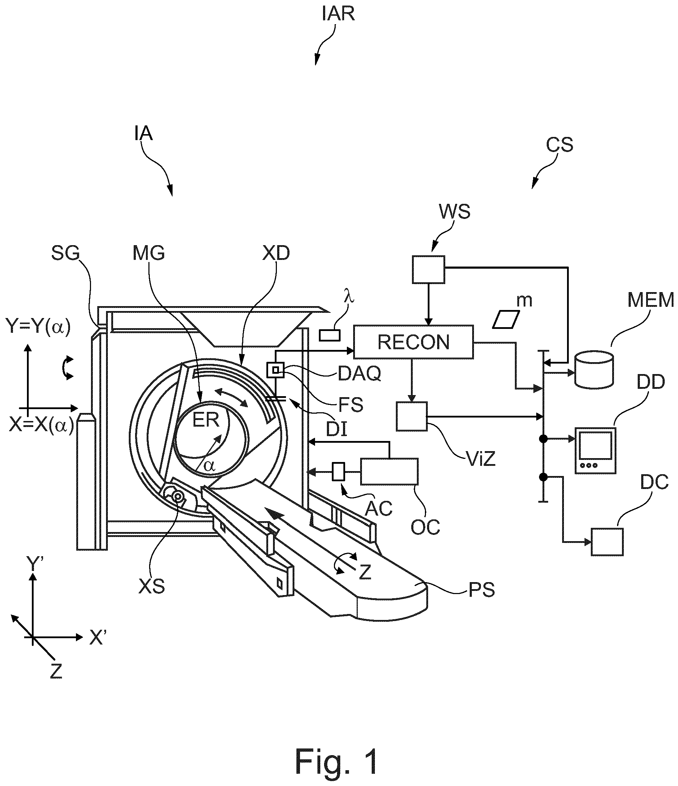

Reference is now made first to the block diagram of Fig. 1 which shows an imaging arrangement IAR as may be used in the medical realm or other.

The imaging arrangement IAR broadly comprises an imaging apparatus IA ("imager"), and an optional computing system CS coupled to the imager IA for processing measurement data $\lambda$ acquired by the imager IA of at least a region or object of interest (both referred to herein as "ROI") within, for example, a patient PAT or other object. The measurement data λ may be so processed into imagery in relation to said ROI, to so better support clinical objectives for example. During imaging at the least the ROI resides in an examination region (a portion of 3D space) of imager IA. The imaging apparatus is preferably X-ray based to include an X-ray source XS from whose focal spot, an X-radiation beam emanates and passes through examination region ER and hence through the imaged object. The radiation, in form of a (relatively broad) X-radiation beam, interacts with matter that makes up ROI, and, as a consequence of this matter vs radiation interaction, is modified. This modification encodes spatial details of internal structure of object, thus allowing imaging. It is the so modified radiation that is detected during imager's operation by its X-ray sensitive detector, arranged opposite imager IA's X-ray source XD, with at least the ROI residing therein in between in the said examination region ER.

Broadly, what is proposed herein is a faster, more efficient operation of the X-ray detector XD, with improved global or average image resolution vs readout speed balance. This is accomplished by a (sub-)system, to be described more fully hereinunder. But before providing more details on the said sub-system, some contextual description is provided first on broader aspects of operation of the imager IA.

The measurement data λ, in general projection data, across the region of interest, is then made available via wired and/or wireless data connection to the computing system CS for the said processing. Whilst projection data in and of itself may be of interest herein, such as for radiography, not excluded herein, it is often computational processing of the projection data that is required first to obtain, for a given purpose, more apt imagery *m.* This may be the case for example in tomographic X-ray imaging, which is mainly envisaged herein in embodiments, and is illustrated in Fig 1 at the example of a diagnostic CT scanner with its characteristic "doughnut" shape, an annulus, with its opening forming the examination regions into which a patient support PS (couch, table, etc.) may extend, with ROI/patient PAT on it. Having said that, instead of such diagnostic type imagers, interventional imagers IA of the tomographic type, such as C-arm scanners, as may be used for image-guided therapy or other, are also envisaged herein.

In such tomographic arrangements, the measurement projection data essentially image data is processed by a computational reconstruction unit RECON which implements a tomographic reconstruction algorithm. This is a mathematical technique that allows converting the projection data (spatial data), acquired in multiple frames from multiple spatial directions α for one or more z positions along longitudinal axis of ROI, into sectional imagery. The reconstructed sectional imagery may include either a single section/slice, or more usual, plural such sections, representing an image (sub-)volume/slab of examination region ER, and hence of ROI in it. Such sectional imagery allows representing a cross-sectional view of ROI to lay bare internal structure of the ROI, now disencumbered from disturbances of intervening structures as in purely projection only based imaging (such as in radiographic modalities). The longitudinal axis is in general parallel to, or indeed coincidental with, imager's rotation/imaging axis Z that is run as, illustrated in Fig. 1, into the examination region ER, and through isocenter.

This imagery *m* so reconstructed from the acquired projection data may be used for various purposes such as to inform diagnosis, therapy, radiation therapy planning or whatever the clinical task at hand. The reconstructed sectional imagery may be passed through wired and/or wireless connection, through bus system, to similar, to a memory MEM, volatile or non-volatile, on which it may be stored. Such non-volatile memory may include a Picture Archiving and Communication System (PACS) in which imagery is stored for later review in a radiological review session by a radiologist, who may then draw up their rad-report. The imagery may be visualized by visualizer VIZ that maps the imagery onto a color or a grey value palette and drives video circuitry to obtain a graphics display which visualizes imagery m on screen of display device DD. There may also be, instead of, or in addition to the above, other data consumer(s) DC, such as artificial intelligence based diagnostic aids for example that process the imagery *m* to localize, flag-up or otherwise indicate possibly suspicious structures that may warrant a closer look by the radiologist etc. Other manner of processing of or application contexts for, such imagery abound, all envisaged herein in embodiments.

The imaging arrangement IAR may include an operator console OC, arranged one or more computing systems, through which operator/user can control operation of the imaging session, to so start, halt or otherwise manipulate acquisition of the projection data λ. One or more actuators AC, such as server motors or others, may allow adjusting imaging geometry to ensure that the region of interest to be imaged is in the field of view of the imager.

A data link D (an interface(s)), such as a slipring interface or wireless link or other, allows passing the acquired data λ from detector XD to the computing system CS for processing. The supporting computing system CS be implemented on one or more computing entities/nodes as mentioned such as a workstation WS that is communicatively coupled to the imaging apparatus, in particular via data output link interface DI and/or in whichever way.

The computing system may be either remotely located or located in the same examination room, may be integrated into the imager IA, as the case may be and as needed. Use of slipring interface datalink or wireless datalink DI allows providing data λ from a mechanically rotational system, such as is often used in tomographic imagers, to the outside for processing, such as by the supporting computing system CS or other.

The imager IA of the tomographic type is configured for multi-directional projection image acquisition. The imaging apparatus IA is thus capable of acquiring projection images λ along different projection directions α relative to the examination region ER, and thus the ROI. Acquisition be done in embodiments by a rotational system where at least the X-ray source XS is arranged in a moveable gantry MG. The movable gantry (and with it the X-ray source XS in embodiments) is rotatable in a stationary gantry SG around the examination region/bore ER in which the patient/ROI resides during imaging. Opposite the X-ray source in the movable gantry there is the X-ray detector XD which may rotate with the gantry and X-ray source around the examination region ER to realize the different projection directions α. Helical or step-and-shot imaging protocols are envisaged herein where there is a relative translational motion along the longitudinal axis Z, between X-ray source XS and the patient PAT. For example, the patient support PS may be advanced through the examination region ER during the multi-directional projection image acquisition, for example during rotation of the X-ray source XS about patient PAT.

The CT scanner setup as illustrated in Fig. 1 is merely according to one embodiment, and other tomographic imaging equipment such as C-arm or U-arm scanners, cone beam CT arrangements, mammographic imagers, etc., are not excluded herein. C-arm or cone beam CT imagers are envisaged herein. The multi-directional acquisition capability may not necessarily result from a mechanical rotational system such as shown in Fig. 1. Non-(mechanical) "virtual" rotational imaging systems are also envisaged, such as in CT scanners of the fourth or higher generation, where multiple X-ray sources are arranged around the examination region in a source annulus for example. In addition, or instead, the detector XD may be arranged as a detector annulus around the examination region. Thus, in such systems there is no mechanical rotation of the X-ray source XS or detector XD, or of both, but by switching source/detector in sequence, a virtual rotation is effected, which also allows multi-directional imaging.

The following spatial 3D co-ordinate system as illustrated in Fig. 1 may be helpful in the following. There is the said imaging/rotational Z axis that extends through the imager's bore ER, and preferably through the isocenter. In a plane perpendicular to the rotation axis Z, the other two axis X and Y span the tomographic imaging axis as indicated to the left of Fig. 1. One of the axis, axis Y, is the imaging axis and in general corresponds to the mean propagation direction (excluding scattering contributions) of the X-ray beam as emitted by the X-ray source during imaging. Perpendicular thereto there is the X axis. Because of the rotation α measured against a reference line that may run in direction 6 to 12 o'clock in a plane perpendicular to the rotation axis Z is a function of rotation angle α. Thus, the imaging coordinate system $(X := X(a), Y := Y(a), Z)$ rotates with the imager IA's rotational acquisition equipment XD/XC. A fixed 3D co-ordinate system may also be used herein which comprises the rotation axis Z and wo fixed axis $X'$, $Y'$ in a plane perpendicular thereto where one axis. such as the $Y'$ axis, is true vertical, whilst the other $X'$ is true horizontal. Such fixed co-ordinate system $(X, 'Y', Z)$ may be referred to herein as the "world co-ordinate system" and can be defined in the examination room as prescribed. For radiographic imaging without such rotation, axes X,Z (now not a rotation axes) span the projection domain.

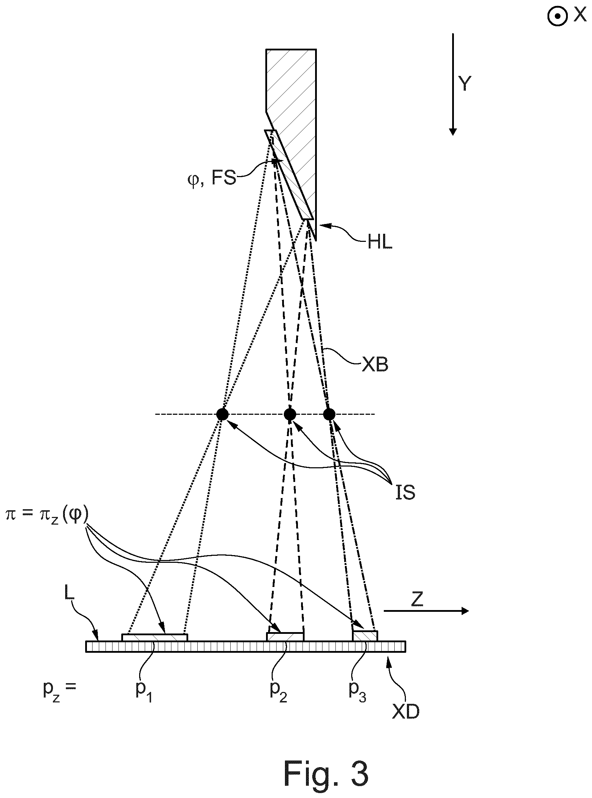

The projection data λ (and the imagery *m* reconstructable therefrom) may be provided in digital form/format, that is, as a set of spatially resolved numbers. The detector XD may be configured to acquire the projection data λ in such digital form natively. A flat panel digital detector module XD may be preferred herein. Such detectors comprise a radiation sensitive imaging plane L (see below at Fig. 3), or more generally an imaging surface L, as curved detectors are also envisaged. The imaging surface L is made up of radiation sensitive pixels which are operative to resolve the impinging radiation, after its passage through the ROI in the examination region, onto the spatially resolved set of numbers. Such spatial digital data can be made available by detector XD on read-out as a matrix or tensor. Such spatial data can be output through the data link DI for processing by system CS, or by any other data consumer(s) DC, as above for tomographic reconstruction and/or otherwise. Detector sided data acquisition circuitry (DAQ), partly or wholly included in the X-ray detector XD, may be operable to process, in particular, convert electrical charges that accumulate in the detector XD due to exposure of the imaging surface L to the X-ray beam, into digital values which can then be processed as described.

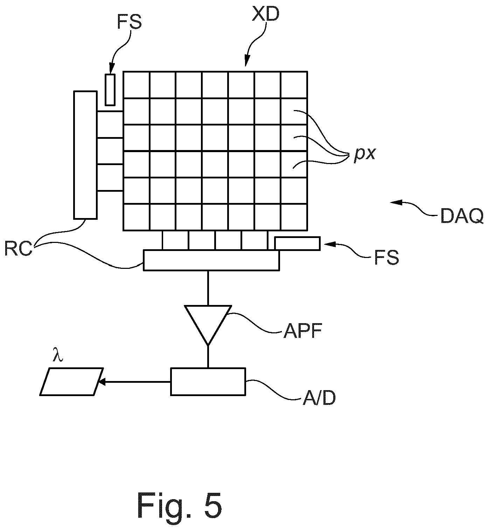

Broadly, what is envisaged herein is the imaging accelerator facilitator FCS (indicated in Fig. 5, on which more below), briefly mentioned above, that allows faster imaging, as may be of consideration in some clinical applications, such as imaging with higher rotation frequency to reduce the likelihood of incurring motion artifacts. It has been found that read-out operation, to be explained in more detail below, that occurs inside the X-ray detector in order to make the projection data available for processing may prove a bottleneck, in particular for high resolution data, as is often acquired by modern detection equipment having particularly small pixel sizes, down to sub-millimeter or tenth of micrometer range. For example, pixel sizes may range from between *50 to 150 microns* for C-arms, to larger pixels (eg, *200μm* to *1500μm*) in CT. the size numbers quoted have example character and are merely for illustrative purposes.

The system FCS balances read-out speed vs average projection data resolution in a favorable way: in places over the imaging surface L where high resolution ("high res") is not needed, some resolution is given up for read-out speed. More aggressive, more encompassing, binning pattern is applied across the imaging surface L at places where there is no benefit (in a sense made more precise below) for high res, whilst a more liberal, less encompassing binning pattern (or no binning at all) is used at other places/locations where there is such benefit. By this judicious balancing, readout speed may be boosted, whilst maintaining a good resolution average over the imaging surface. Thus, a given read-out projection frame in generally has spatially varying resolution. But "patches" where there is lower res than detector XD's native resolution, are favorably distributed. The average resolution globally across the imaging surface L is sufficient for the medical imaging task at hand.

More specifically, but still broadly, the read-out boost facilitator system FCS (referred to hereinafter for brevity as the "facilitator system FCS") can be understood as a read-out booster system FCS that allows, for a given average resolution over detector XD's surface L, faster read-out by applying locally adapted pre-read-out binning scheme/pattern, that combines data acquired by the detector pixels into chunks that can then be read-out per chunk rather than individually per pixel. This causes much better read-out rates which can be useful for imaging as the rate of imagery having sufficient quality can be increased. Faster readout can be used to read out more data or to reduce the scan time, which reduces image artifacts due to patient motion. Conversely, in high res imaging, binning can help increase the field of view. A larger field of view can be covered for the same read-out cost as a high res scan for a smaller FOV. This may be useful in CBCT, such as in cardio applications, or other, in particular, but not only, for interventional imaging (C-ram, U-arm of similar).

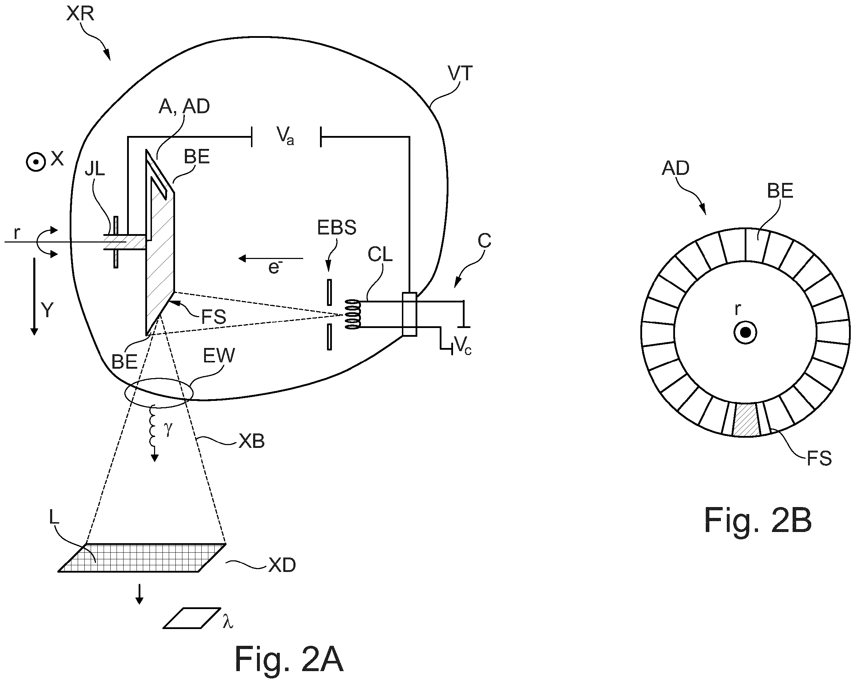

Reference is now made to Figs 2A, B which lays bare more details of imager IA's acquisition equipment, in particular of the X-ray source XS component thereof. The source XS may comprise an evacuated vacuum tube VT, possibly done from glass, arranged in a housing (not shown). Inside the tube VT there is an anode A and a cathode C. The anode A, shown in cross sectional view in Fig. 2A, is arranged as a disc rotatable about rotation axis R, the disc A suitably journaled JL to this effect. Such rotation affords better heat management as the disc is subjected to considerable temperature. This is because, in operation, an electron beam $e^-$ impacts anode disc A at high velocity at its focal spot FS. The focal spot FS of anode (disc) is located, relative to the world coordinate system, on a beveled edge BE. Upon impact there, the electron beam $e^-$ is decelerated and excess energy is partly released as an X-ray photons $\gamma$ that, thanks to beveling BE, issues forth from the focal spot FS, in particular in a mean propagation direction of the released portion of the X-ray photons $\gamma$ to form the beam XB. Said mean propagation direction of released X-ray beam XB is approximately perpendicular to the mean propagation direction of the electron beam $e^-$. The beam of X-ray photons $\gamma$ form the X-ray beam XB are so released through an exit window EW of housing, to then interact with patient tissue and X-ray beam is then, as was briefly described above, incident on the X-ray detector XD to generate the projection data, as illustrated at the foot of Fig. 2A.

The acceleration of the electron beam $e^-$ is caused by accelerator voltage $V_a$ that is applied during operation across the anode AD and the cathode C. The cathode C essentially serves as a supply of the electrons that are bubbled off a coil CL or grid structure, are then released in the vacuum and are accelerated by voltage $V_a$ at high velocity towards the anode disc AD for impact there at focal spot FS as describe above. Through the cathode C coil CL or grid a current is applied driven by cathode voltage $V_c$ to heat up coil CL, thereby causing the bubbling-off of the electrons $e^-$ for acceleration towards the anode disc AD. An electron beam shaper EBS may be operative to shape the bubbled off electrons into the electron beam $e^-$ for better focusing on the anode disc AD. The electron beam shaper EBS may be configured from higher density material as claws to form an aperture through which some of the electrons escape to form the electron beam $e^-$. In other embodiments, the electron beam shaper EBS is a grid, which is electrified with a negative charge, to force, thus form, the electrons into the electron beam $e^-$. In yet other embodiments, the electron beam shaper EBS may include two or more electromagnets that operate to generate a magnetic field through which the electrons pass, thus forming them into the said electron beam. The strength of the current through the emitter coil CL and the strength of the accelerator voltage may determine the energy of the photons $\gamma$ that make up the X-ray beam. a larger accelerating voltage Va may cause a beam of higher energy, as may be called for by certain protocols, in particular when imaging a thicker object. The intensity of the X-ray beam is controlled by the current at the emitter. Otherwise, a low signal to noise ratio will result as too few photons may be able to pass through the imaged objection (eg, patient tissue) to reach the detector XD, due to their being attenuated by the object and due to scattering or other matter vs radiation interaction effects.

Fig 2B affords a view along the rotational axis R in the direction from cathode C to anode A, AD, just like the electrons $e^-$ travel. As schematically shown there, the focal spot FS is unlike a point, more like a spatial 2D region on the anode surface, owing to some divergence of the impacting electron beam e-. Indeed, focal spot FS has an area of definite height (along Y) and defined width (along X) and hence shape. The shape will depend on the particulars of the electron beam $e^-$ that are impacting the anode disc surface. An approximation quadrangular shape may be assumed as schematically indicated by hatching in the Figure. Local co-ordinates of the focal spot on the disc are changing of course due to the rotation of the disc, but in terms of the world co-ordinate $(X', Y', Z)$ the focal spot, the $e^-$-irradiated region, remains stationary.

Reference is now made to Fig. 3, which shows, inside elevation, the x-ray imaging mechanism, that is, interaction between the x-ray source XS, and the detector XD in more detail. In particular, it is shown how the x-ray beam XB issuing forth from the anode disc, in particular from the focal spot FS, impacts the imaging plane L of the X-ray detector XD.

The view is perpendicular onto the plane spanned by imager's rotation axis Z and projection axis $Y$ of imager IA for a given rotation angle $\alpha=0°$, but the following is applicable for any such angle. As mentioned in Fig. 2, the focal spot FS is not a geometrical point but has area and shape, in short, spatial extent, to form a portion/region, referred to herein as face $\varphi$ of focal spot FS. That face $\varphi$ appears in projection view $\pi(\varphi)=:\pi\varphi$ differently as footprints at different pixel locations pj, depending on the projection lines cast from focal FS to such respective locations. For illustration, three such pixel locations are shown p1-p3, with their respective projection directions shown in dotted, dashed and dotted-dashed bundles of lines. In the particular situation illustrated in the Fig. 3, such projections amounts to "downwards" view for the example angle $\alpha=0$, but may be upwards at 180°, and accordingly for other angles $\alpha$, the particular angle being immaterial for present purposes. The focal spot has a "native" face $\varphi0$ (not shown) with native/true spatial extent, as would be revealed if one were to look at the anode disc along the normal of its surface at the beveling BE where the focal spot forms. However, under projection $\pi\varphi(p)$ onto the imaging surface L, this native face is subject to perspective distortion so that its effective face $\pi\varphi(p)$(its projection footprint, or, for brevity, simply "footprint") as recordable on imaging surface L is a function of pixel position p. This function or such functional relationship $D: p \rightarrow \pi\varphi(p)$ may be referred to herein as the focal spot face distorter function $D()=D,$ or as "distorter function", again for the sake of palpable brevity. Thus, x-ray beam XB impacts pixels p1-3 on the detector's plane L differently when such projectional/perspective distortion is accounted for as proposed herein. The spatial extent over L of focal spot's face $\pi\varphi$ will be different to different pixels p$j$, such as p$z$ along Z. For example, an inlying pixel p2 that is situated vertically downwards from a center point of the native focal spot face Φ0 will receive radiation in proportion to the spatial extent of the phase Φ0 as it appears in that particular projection direction. Moving to the left or right along a line in image domain, such as along the rotation axis Z, that is, moving towards or away from the anode disc's heel HL, the focal spot phase will appear smaller or larger. For example, at pixel p3 situated beyond or towards the heel from "inline" position of p2, the footprint will appear smaller in projection view than it does at P2, whilst at position p1, away from heel HL, there will be larger footprint recorded on radiation. Thus, the spatial extent of footprint depends on the pixel position. That spatial extent may be affected in terms of size of footprint as illustrated, but also/or instead, geometrical shape of footprint as project onto L. This distortion of spatial extent as projected impacts the measurement signal as recordable at a given pixel, as the intensity that he pixel ought to register is spread differently as per the distorter function D. Along any line (such as along X or Z or any other) parallel to or on the surface L, the distorter function D varies with position along that line, such as for p(z) in the Figure. The focal spot's projection footprint may not necessarily vary with every pixel position px (although it may well do, at least along some direction(s) in the surface L). In general, the particulars may depend to the focal spot size, detector's pixel pitch, and other imaging geometry particulars.

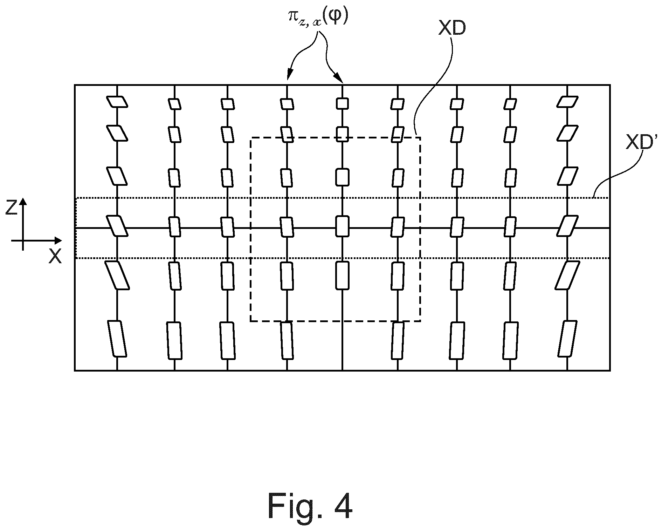

This effect, or the distortion function D, is also illustrated in Fig. 4. Thus, Fig. 4 affords a protectional view from the x-ray source (here, a rectangular shaped face of focal spot) onto the surface L, and thus as view on how the focal spot phase footprint would appear on the imaging surface L, as a function of a position p on L or along various lines in image 3D image domain (the 3D space between source XS and detector XD) more generally. For example, there is perspective distortion of the spatial extent of the focal spot not only along Z direction as indicated earlier in Fig. 3, but also perpendicular thereto along direction X. Thus, for example for a CBCT detector, roughly of square shape whose demarcations are shown in dashed lines, has negligible distortion in direction X, but more so in direction Z. The situation is the inverse in CT detectors that are relatively long in Z direction, but relatively short in slice thickness direction along Z direction. For such so elongated CT detectors or other such detectors or modalities, the distortion is more prominent along the X axis, perpendicular to the rotational axis Z.

What is then proposed herein, and as will be illustrated with Figs 5-6, the proposed facilitator system FCS uses a locally adapted bin operation with a specifically binning filter, or (convolution) kernel $\kappa=\kappa(p),$ with such adaption a function of position/location $p$ on L. The binning kernel equally has spatial extent in the detector plane L when operative to combine, such as convolve, signals across plural pixel positions $p_j$ in general. What is proposed herein is that the spatial extent then of a read-out binner's kernel $\kappa$ varies with pixel position $p$ in the same (or more generally, corresponding) manner as does the projection footprint of the focal spot's phase varies across those pixel positions $p$. That is, and as was illustrated above in Fig. 4, some locations p on the detector plane/surface L will experience no binning, that is the pixels there are read-out as is, whilst other pixel positions that receive a more distorted view on the focal spot's face, will receive a binning based on an accordingly shaped 2D binning kernel. Thus, it can be seen in general that the larger the size of the projection footprint of the focal spot, the more encompassing binning there is there, that is, more pixels will be combined there under such circumstances.

For example, referring back to Fig. 3, the local target pixel position p1 will receive, that is, will be processed with, a larger binning kernel to combine more pixels in its neighborhood, than does pixel p3. And p2, may see no binning, thus, is read-out as is due to absence of distortion. Other pixels may experience binning in between the three extremes, may be a case in-between. Interestingly, it is not only or not always the size of face footprint πφ that is changing across the detector plane L, as illustrated in Fig. 4, but it is also, or instead, the shape of footprint πφ that changes. In the latter case, the proposed binning kernel varies in its shape accordingly, but not in size. In general, it is footprint πφ's shape and size that is varying with position *p*. Thus, at some locations p on L, there may be more spatial extent in the X direction than there is in the Z direction. And in this case, more pixels in the X direction will be included in the combination for any given pixel position than being combined perpendicular to and vice versa. In either case, the face distortion dependent kernel adaption allows better, because more targeted, harnessing of the benefits of binning: it allows reducing the work done by the detector's read-out circuitry, and yet allows more realistic projection imagery to obtain, where higher resolution is awarded where it is useful, and sacrificed where it is not. The read-out speed vs resolution tradeoff or balancing is played out herein in finer spatial nuances, because it the proposed adaptive binning strategy is capable of following both, shape and size changes of footprint as function *D* of position *p* on L in either or both spatial dimensions X, Z. For example, at position p3 (Fig. 3) in direction Z, less binning is needed, compared to position p1, where a larger resolution there would be of little gain as the same signal is spread around a larger area of pixels on a particular shape. Thus, shape and/or size of the spread are preferably both are taken into account with the proposed dynamic binning kernel adaptation.

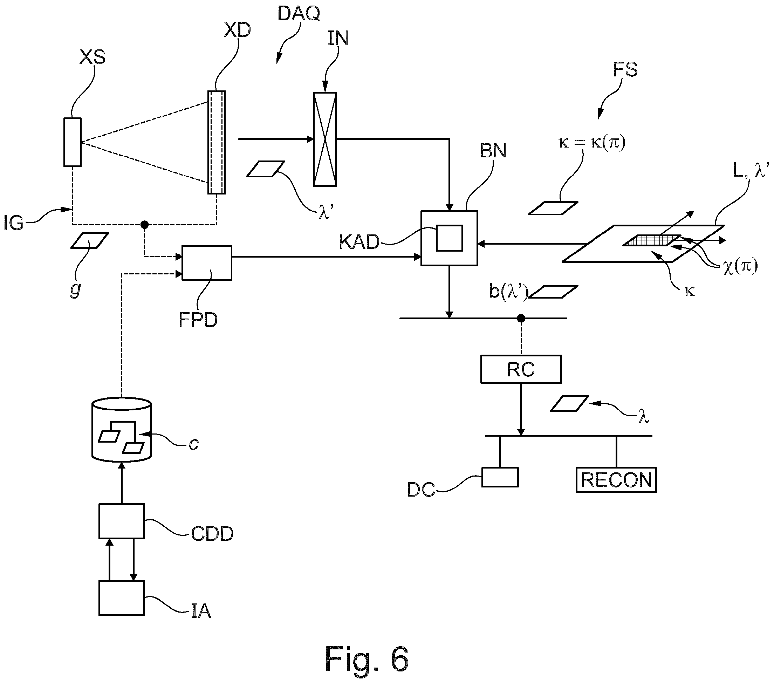

Operation of the binner and its binning kernel is now explained in yet more detail with continued reference to Figs 5, 6.

First, with more detailed reference to Fig. 5, this shows the detector plane L in plan view, along the imaging or mean projection direction axis *Y*. The detector plane, more generally surface, L is made up of pixels px at different pixel positions *p* across L, spatially arrangeable, preferably, in a 2D pattern, such as a matrix fashion.

With more detailed reference to the Figure, this illustrates schematically components/circuitry of the X-ray detector module XD. Such components may include the radiation sensitive surface L formed by pixels *px,* arranged in a matrix layout as shown in plan-view in Fig. 2. The pixels px may be arranged as transistor components that accumulate charge λ' in proportion to dose exposure. Direct or indirect conversion designs are envisaged for detector module XD.

The detector module XD may include general operational control circuitry (not shown) and the said read-out circuitry RC, arranged for example at and along the sides of the pixel matrix. Such circuitry RC allows reading out (electrical) charges accumulated at transistors that are used to form the respective pixels. The said charges λ' are due to X-ray exposure. Controlled by control circuitry, individual pixel positions in the detector matrix *p(x,z)* can be read out by drain lines, along which a respective charge is collected, integrated over time by integrator circuitry (not shown) and may then be output (read -out) by read-out circuitry RC. The mentioned over time (t to *T*) integration by integrator is done to obtain

fluence from flux, $\Phi_p = \int_t^T \varphi_t^p \, dt$ and it is respective fluence that is encoded at the respective pixel *p*. Notwithstanding the above, other detector technologies, such as photon-counting detectors are not excluded herein.

The read-out charges or currents may be conditioned, such as by shaping, or amplification by amplifier APF. The analog domain signal in form of the charges (voltage) or currents (amperage) λ' so obtained may be converted into digital format by an A/D converter. The charges or currents held in pixel circuitry form pre-readout frame *λ'*. The illustrated position in the detector XD's processing chain of A/D unit is for illustration. Instead of to what is shown in the Figure, A/D unit may be located upstream read-out circuity RC.

Thus, as illustrated in the Figure, upon x-ray irradiation, the pixel PX circuitry receives a spatially distributed signal λ' which is then collected, after system FCS's binning, by the read-out circuitry RC, and may be passed onto the next stage in the processing chain, until projection data is delivered to consumers DC, RECO, visualization, for storage, etc. λ will be thus referred to herein as post-read-out projection data/imagery, whilst λ' as the pre- read-out projection data. The facilitator system may operate in analog domain upstream aid conversion stage (not shown) or in digital domain downstream aid conversion in the DAQ processing chain. Thus, system FCS may operate (in particular by binning, or by causing such binning) in analogue or digital domain. Thus, the conversion into digital domain may occur before or after read-out.

DAQ processing chain may include further signal condition, such as filtering, amplifying, the said time integration, etc, in addition to said A/D unit and readout operation by circuity RC. The facilitator FCS operates upstream the readout-out circuitry RC. Facilitator FCS accesses pre-readout data λ' and produces binned data b(X'), which is the deliverable post readout projection data λ. (Post readout) projection data λ may be further processed such as ald-ed, signal conditioned, etc by some or all of the said other DAQ components operate upstream facilitator system FCS, or downstream thereof. Thus, the facilitator system FCS is preferably located as indicated upstream of the read-out circuitry RC and is operable to combine the accumulated charges or currents or signals (representative of λ') according to the spatially adapted (in shape and/or size) kernel κ(p) as a function of p∈ *L.* As a matter of principle, once pre-read-out data λ' is accessed through suitable pick-up circuitry, the binning operation as such (the combining of the pixel charges λ' into chunks) can be done away from the x-ray detector XD. However, following edge computing principles, the combinational binner functionality may be located closer to the data source (in this, the detector XD), such as on in-between the pixel px circuitry, and the read-out circuitry RC, as shown in the Figure.

As will be appreciated from the above, the higher the spatial resolution, the more time latency is incurred due to integration and read-out work needed per pixel. The more pixels per frame, the longer the readout time.

The block diagram in Fig. 6 shows yet more details of the facilitator FCS's operation. Pickup input circuitry IN accesses the distributed charges λ' at pixels pixel px, prior to read-out at read-out circuitry RC. At each targeted pixel *p,* that is, at each pixel *p* at which a binning operation is to be performed, an adaptive binner BN uses a spatially adapted kernel $\kappa(\pi\varphi(p))=:\kappa(p)$. Local 2D $\kappa(p$ corresponds to distorter function D(p) at p. For example, $\kappa(p)$ is adapted in spatial extent on proportion to projectional/perspective distortion of the footprint that would appear at that target position p. This was illustrated above at Figs 3-4. Thus, depending on an applicable kernel adjustment policy, spatial extent of kernel $\kappa$ is adapted to correspond, eg conform with, spatial extent of projection footprint it appears in the detector plane L at the respective *p.*

Such spatial extent specification $\chi$, such as the extent in X,Z direction, may be stored in a suitable data structure, memory register. They are queried by binner BN and applied to a neighborhood around, and usually including, the target position p. Thus, charges at pixel coordinates as specified by the locally adapted kernel $\kappa$ are then combined and provided at output OUT as the combined pixel values b(Z') which can then be read-out by the circuitry RC and provided, as the binned image values at for locations p of L. Such binning occurs in all or some of the locations *p* across the image plane L.

Depending adjustment policy as administered by kernel adjuster KAD, some pixels that receive a view on the focal spot's phase with no or small distortion, may experience no binning. For example, pixels in regions that receive a view on spatial extent distortion *D* lower than pre-defined threshold *T,* receive no binning at all. These are in general pixels that are located close to the intersection with L of the perpendicular line that may be dropped on L from a reference point of focal spot FS's face $\varphi$, such as its center point, or other.

The spatial extent $\chi$ of the applicable binning kernel K may be provided by a projection footprint determiner FPD or spatial characterizer that characterizes spatial extent $\chi$ of footprint $\varphi(p)$ and thus of kernel $\kappa(p)$ at p. Footprint determiner FPD essentially determines or samples distorter function *D(p).* Based on such data *D(p),* kernel adapter KAD then provides, that is, adapts, a respective kernel $\kappa(p)$, and this is then applied by binner BN at some or each *p* in *L*: $b(\lambda') =\{(\sum_{p'\in\kappa(p)} \lambda'(p'))_p\}$, *p'* being positions in the neighborhood of *p* (including p itself) as per the set of pixel coordinates $\kappa(p)$ mapping out the applicable spatial extent in *L* as per *D(p).*

At least two embodiments footprint determiner FPD are envisaged herein: in one, footprint determiner FPD is based on a classical geometry machine. It uses that uses imaging geometry specification g gathered from the current imaging geometry IG. Such imaging geometry data include as the size of L, native pixel positions, the source detector distance, etc. Based on g, footprint determiner FPD uses analytical projective geometry algorithms, relations, formulae, to compute the exact spatial extent of the projection footprint $\pi\varphi(pj)$ of the focal spot FS at each given pixel position *pj.* The computed regions may be provided as regions enclosed by curves in L. Such regions/their curves may be suitably discretized by a discretizer (not shown) of footprint determiner FPD. The respective pixel co-ordinates are noted/stored in a suitable data structure (LUT, associative array, matrix etc), in association with the respective pixel position *pj.* Thus, the footprint determiner FPD provides a kernel blueprint for some or each pixel position *pj.* The blueprint may be understood as a collection of such pixel positions per target *pj,* which are then used by kernel adapter KAD to adapt kernels per p, which are then applied by the binner BN in combining the respective charges around, ad in including, the charge at the given target pixel *pj.*

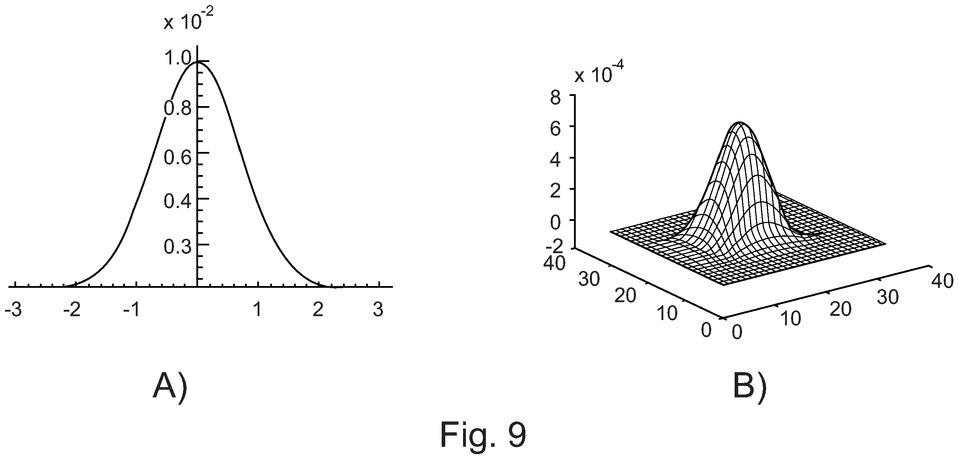

In another experimental embodiment, calibration data c may be used instead of or in addition to, the geometric theoretical approach above. Calibration data may be stored in a memory MEM'. A calibration data determiner CDD is operable to interact with the imaging apparatus IA in a calibration phase to acquire test images at respective pixel positions of what is essentially a sampling of the local point spread function or MPF for some or each p in L. Imager IA may be operable to produce pencil beams in good approximation, and the local images are then measured per pixel position and stored. The recorded spatial extent of the respective point spread may then be discretized into a respective pattern $\chi$ (eg, sub-matrix of L) of pixel positions that represents the applicable kernel $\kappa$ at that p. An edge segmentation algorithm, based on Canny or Sobel filter, or any other edge segmentation algorithm may be used for this. The discretization may be done by fitting a maximum primitive, such as a quadrangle, along X and Z into the segmentation $\chi$, to obtain the pixel coordinates of kernel for a given target pixel. Alternatively, and more refined, a grid is fitted into the segmentation with possible fractional binning at its edge, where only a corresponding fraction of the charge at the edge pixel is combined into by kernel. The described discretization may also be used in combination with the analytic geometry embodiment above, to so discretize into a pixel pattern for use as kernel the respective analytical closed curve representation for the footprint $\pi\varphi(p)$. As said, fractional binning of pixel contribution may be used in some embodiments. Further details on calibration may be provided later, below, at Fig. 9.

The binned data $b(\lambda)$ is then processed by the read-out circuitry into the deliverable projection data $\lambda$. The deliverable projection data $\lambda$ may then be passed through the output interface DI for processing by the data consumer DC, such as for reconstruction, storage, and/or whatever manner or processing, application, use or purpose.

As briefly mentioned above in connection with the analytic geometry embodiment, the spatial characterizations, such as their respective spatial extent $\chi$, is a function of the imaging geometry IG, g to be used. Thus, the spatial extent $\chi$ is a multi-dimensional data set $\chi(g)$ as a function of $g$ for any of the embodiments, not only for analytic geometry embodiment but also for the experimental /calibration-based embodiment. It may be sufficient at times to sample $\chi$ for certain $gj$'s, store the results $\chi(gj)$ in a LUT or similar, and then interpolate for a requested imaging geometry g specification from 'sample/reference specifications $\chi(gj)$.

As mentioned, different kernel adjustment policies may be used by kernel adjuster KAD. Sometimes, at location, footprint shape changes relative to native true footprint $\varphi0$ , but not its size. Or it is size that changes, but not shape. Or it is both, shape and sizes that changes for certain p's. In any case, the kernel filter is the larger, the larger the size of the footprint under distortion. This allows saving valuable read-out work for locations p where signal is spread out widely. But even if size remains, a spread into one direction or other by shape change, for example when a square is elongated into a strip, in, say, direction z, the proposed binning allows capturing this shape distortion effect. Read-out work in z direction is then useless and can be saved, whereas in x direction it may be worthwhile, etc. As per one such policy, $\kappa(p)$ changes in same manner as $\pi(\varphi)$ to directly map the spatial extent to $\kappa$. Alternatively, it is the relative change of native $\varphi0$ vs distorted $\varphi$ that is considered, and variation is based one relative proportion of $\chi(\varphi0)$ vs distorted $\chi(\varphi)$, $\chi$ being the spatial characterization such as shape or area. An operational tolerance ratio $r = \chi(\varphi0)/\chi(\varphi)$ may be formed and considered to guide operation of adjuster KAD. Some p's see no binning at all, for others the binning is in proportion to the amount by which the ratio $\chi(\varphi0)/\chi(\varphi)$ [p] at given p exceeds tolerance ratio $r$. The policy may be predefined, or user adjustable through GUI or other UI. Thus, clinical end user, or system engineer at the manufacturer side, may be so enabled to tweak ratio $r$ for example.

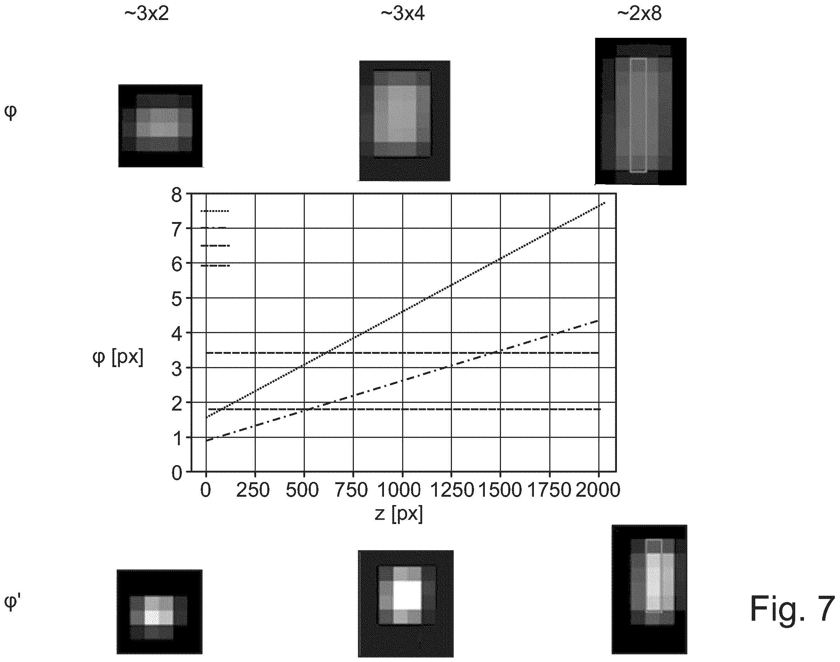

Fig. 7 shows projection view of the kernels of different sizes (in number of pixels), such as *3x2, 3x4, 2x8,* as shown in the top part of the Figure from left to right for different focal spot faces $\varphi'$, $\varphi$, the upper one $\varphi'$ being larger than the lower one in spatial size. Thus, the top one *"IEC07"* may belong to a focal spot with a nominal focal spot value of *f=0.7* (IEC 60336) specification, whilst the lower one *"IEC04"* belongs to a focal spot with a nominal focal spot value of f=0.4 (IEC 60336), for example.

The two vertical dashed lines show the number of binned pixels in the X direction for the two focal spot sizes as a function of the pixel position along the Z-direction. The two dashed and dotted lines show number of binned pixels in the Z direction for the two focal spot sizes as a function of the pixel position along the Z direction for a CBCT detector as illustrated in Fig. 4. As can be seen, for a CT system, the footprint of focal spot face (and hence the applicable kernel) increases by about a factor of 4 as a function of pixel position $p$ along the z-direction (2000 pixels) in the detector center. The footprint of focal spot along the x-direction is almost constant.

In C-arm systems, the adapted binning can be used to increase the area covered by the kernel by about a factor of 2 relative to the native focal spot footprint of a focal spot of specification, with almost no loss of resolution. This may enable *VasoCT* or high-resolution stent protocol acquisitions with double the FOV size. For a larger focal spot size (e.g. IEC07), a binning kernel size extended by factor of 2 in x-direction, and by a factor of 2 in z-direction for pixel number 1000-2000 reduces the number of readouts from *2,000\*2,500=5,000,000* to *(1,000+500)\*1,250 = 1,875,000,* a reduction of about *38%,* with only a small loss of resolution.

The slanted lines dotted and dash/dotted indicate the spatial extent as it appears with increasing distance along the Z direction each taking-in pixel units *px.* To the right at kernel size 2x8, the solid rectangle shows a suitable discretization of the pixel co-ordinates for the kernel so be used for the respective pixel position. The Figure illustrates calculated and simulated sizes and shapes of footprints of focal spot faces in units of detector pixels, for two relevant focal spot sizes as used in CBCT, and a pixel size of ~ 150 $\mu$m. The simulated and calculated footprints evidence of a good match in size and shape.

As disclosed herein, depending on the particulars of the focal spot (such as IEC04 and IEC07 or other specification), different binning schemes can be applied in z -direction. In x-direction the same insights as for CT can be applied.

Any of the binning embodiments described herein can be combined with yet improved binning scheme (fractional binning), which adds only fraction(s) of recorded pre-read out data pixels to the resulting binned pixels, for example by combining the data of 3 pixels into 2 pixels or 5 pixels into 3 pixels, etc. By fractional binning, a yet better match of the binning to the effective focal spot size can be achieved.

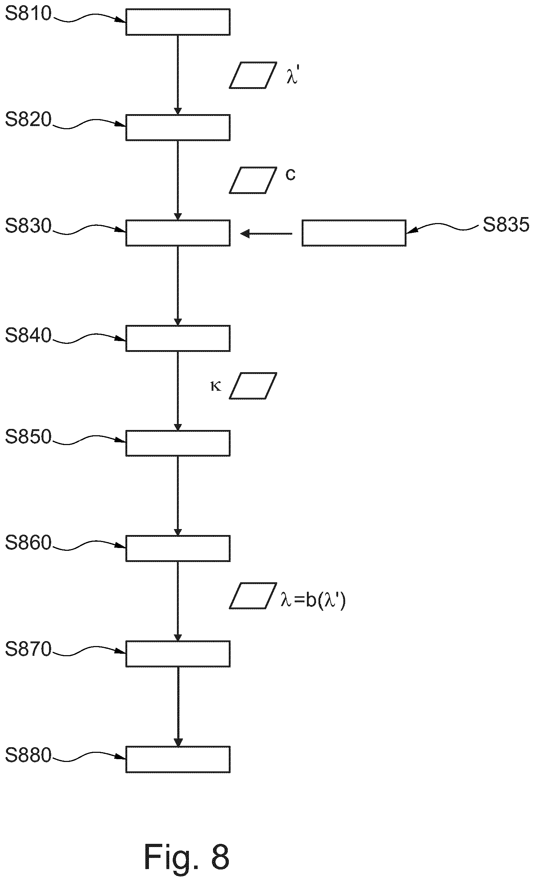

Reference is now made to Fig. 8, which shows a flow chart of a computer implemented method as may be used for x-ray imaging, in particular for read-out binning as may be used herein in embodiment for an x-ray detector.

At step S810, the detector is exposed to x-radiation issuing forth a focal spot of an imager's X-ray source, whilst the patient or at least the region of interest, resides in imager's examination region, to so acquire

projection raw data $\lambda'$ as a set of spatially distributed charges that at this point accumulate in the detector circuitry, in accordance with radiation intensity as captured by detector's pixels circuitry.

At step S820 such projection raw data $\lambda'$ (charges or (amperage) or in whichever form) are accessed or picked up prior to read-out by suitable interfacing with pixel circuitry.

At step S830 it is established for some of each ("target") pixel position as accessed at step S820, a respective spatial extent characterization of binning kernel to be used by a binning operation to be applied at the respective target pixel's location. Such spatial extent characterization may be imported from a data structure or from a registry, memory, LUT, or from any other data source. The spatial extent characterization is based on the spatial extent of a face of the focal spot as it appears under projection along a respective projection line that may be cast from focal spot to the respective pixel position on detector's imaging surface that is made up such pixels. Thus, the spatial extent spatial extent characterization of binning kernel varies with (that is, is functionally dependent on) pixel position, and there is such (possibly but necessarily different) spatial extent characterization of binning kernel per pixel. The spatial extent may be used on geometrical calculations based on the imaging geometry to be used. Alternatively, of on in addition, spatial extent is determined at step S830 is based on calibration data obtained in calibration operation S835 based on prior recoded data at the imager's detector.

At step S840, the binning kernel is adapted based on the spatial extent characterization per pixel to so obtain for some or each such pixel an associated binning kernel. Thus., the proposed setup is a locally (per pixel position) adaptive binning strategy, with spatial extent in the imaging surface of the respective binning kernel that varies with the projection footprint of focal spots face as may appear at the said respective pixel. Spatial extent includes shape and/or size as projectable onto the imaging surface of said footprint and the kernel.

The binning per pixel is then applied at step S850 to combine charges in the neighborhood of that respective pixel, included in the charge at the target's pixel, in spatial correspondence with the spatial extent of the binning kernel.

The binning operation S850, possibly using different kernel with different spatial extent at different pixel position, may be repeated at some or each pixel position until the binning operation has been applied across a portion or the whole of the image plane/surface L of the detector for the given frame.

The spatial extent of the locally adapted binning kernel can be established S830 on the fly, but is preferably pre-computed, stored and may then be retrieved at or during binning operation. The local projection footprints of focal spot and hence of the spatial extent characterization of the respective local kernel may be function of the particular imaging geometry used. Imaging geometry pertains to the mutual spatial 3D configuration between source and detector. Thus, imaging geometry adjustments may call in general for different set of such spatial extent characterization, and hence kernels.

The above steps of accessing the respective position S820, and the establishing S830 or obtainingS840 of the applicable spatially adapted kernels can be interchanged. The establishing step S830 may be based on analytical geometry calculations or on prior obtained point spread function-(PSF)-based measurements, obtained with test imagery without imaged object present in-between source and detector. For the kernel adaptation S840, different adaptive/adjustment policies may be used, based tolerance thresholding, distortion ratios, etc. In general, the kernel adaptation S840 may be based on the local distortion function $D(p)$.

At step S860 the binned projection data $b(\lambda)$ is then made available and can be read out by read-out circuitry at step S870 of the detector.

At step S880 the read-out projection data $\lambda$ is then made available for down-stream processing such as reconstruction, storage in memory or visualization on the display device as needed.

Figs 9A, B illustrate data in connection to the above-mentioned calibration operation S835 to establish the spatial extent of the applicable kernel at the given position. In detail, the respective spatial extent (shape and size) of the kernel $\kappa(p)$ at a given $p$ can be determined by measurements, simulation, and/or calculation of the PSF function (as approximation of the distorter function $D$) for each region of the detector, for a given focal spot face shape and size. For example, in the earlier mentioned calibration based, experimental, setup an array of small metal spheres (or of other essentially radiation opaque material) is placed at the isocenter of the imaging system IA, and detector signals are measured on X-ray exposure. By using such a relatively small objects (phantoms), such as balls, of high attenuating material, the radiation shadow (umbra) that such small test objects may cast upon irradiation may be used to estimate tiny local the point spread function. Small focal spot gives a small shadow and a large focal spot a large shadow.

Each signal of a given metal sphere will be smeared out about a given position p, and will approximate a gaussian like 2D distribution (see Figs 9A, B) on the detector's surface L. For each region or pixel, a value is determined eg, by processing the PSF at p, for example by combination to characterize spread as per PSF around a given point by a measure, statistical or otherwise. For example, FWHM (full width eg, at $q\%$ of maximum of the point spread function in $mm$) metric is taken, such as for $q=20$ or other, along each of X and Z (detector coordinates) to obtain metric values $Vx$ and $Vz$ per pixel $p$ or region $\subset$ L. These two metric values $Vx$ and $Vz$ may be divided by the pixel pitches (which are known from detector's specification) in A and Z direction, respectively, to so calculate the respective numbers $\#X(p), \#Z(p)$ of pixels in x and z direction to be binned. This yields a discretized approximation of the focal spot face footprint at/around a given pixel p, and hence a discretized approximation of kernel $x(p)$'s shape and/or size (that is, its spatial extent $\chi$) in form of a sub-matrix $\{\#X(p)\} \times \{\#Z(p)\}$ (wherein "$\times$" denotes as the cartesian product of sets applicable pixel coordinates) that defines $\kappa(p)$. In the above, $Vx$ and $Vz$ are two continuous maps $Vx==Vx(X,z)$ and $Vz==Vz(x,y)$.

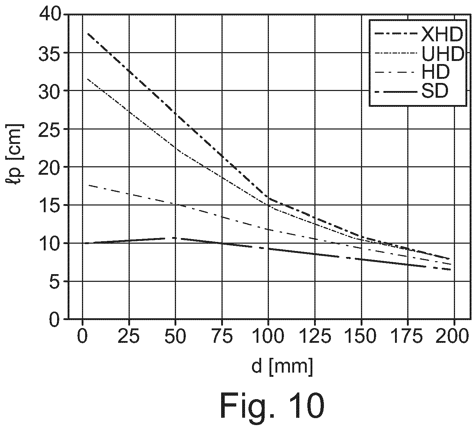

Fig. 10 illustrates effects of decreased spatial resolution in CT images by larger pixel sizes. Specifically, the Figure illustrates the resolution in terms of MTF (modulation transfer function) at 10% of an axial CT image for a small focal spot size and different pixel sizes. The horizontal axis is the distance from the isocenter in *mm,* and the vertical axis is number of line pairs (lp) per *cm* detectable at 10% MTF. The various curves relate to different resolution specifications of the X-ray detector, such as extra high definition (XHD), Ultra High Definition (UHD), High Definition (HD), and standard definition (SD). For SD pixel size ($\sim$ 1.5 mm $\times$ 1.0 mm), the spatial resolution is almost constant, and no benefit of the small focal spot size is gained. At large distances d from the isocenter, the resolution is almost independent of the selected pixel size and a larger pixel size would provide almost the same image resolution as the smallest pixel size, demonstrating the applicability and usefulness of the proposed binning scheme.

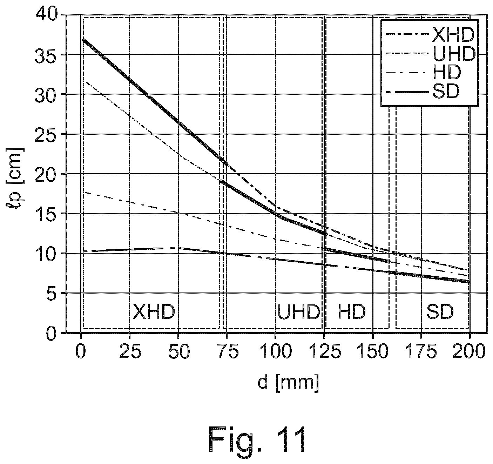

Fig. 11, having the same axes as in Fig. 10, illustrates graphically the proposed binning scheme at the example of CT detectors of different layouts L.

Components of system FCS may be implemented as one or more software modules, run on one or more computing nodes associated with the imager IA. The system FCS may be integrated at least in part in the operating console, or indeed partly or fully into the detector module XD.

Alternatively, some or all components of the system FCS, in particular the IQ assessor IQA, may be arranged in hardware such as a suitably programmed microcontroller or microprocessor, such as an FPGA (field-programmable-gate-array) or as a hardwired IC chip, an application-specific integrated circuitry (ASIC), integrated into the imager IA in particular its detector XD. In a further embodiment still, the system FCS may be implemented in both, partly in software and partly in hardware.

The different components of the system FCS may be implemented on a single computing unit. Alternatively, some or more components are implemented on different computing units, possibly remotely arranged in a distributed architecture and connectable in a suitable communication network.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. System (FCS) for X-ray imaging, comprising:-

    input interface (IN) through which is receivable detector data detectable by an X-ray detector (XD) of an X-ray imaging apparatus (IA) upon irradiation by X-radiation from a focal spot (FS) of the imaging apparatus (IA)'s X-ray source (XR); and
    a binner (BN) capable of binning the detector data into binned detector data, and wherein the said binning by binner (BN) is based on a manner in which a spatial extent of a projection on the detector (XD) of the focal spot (FS) is changeable along at least one spatial direction (X,Z).

2. System of claim 1, wherein the said change in spatial extent is a result of perspective distortion.

3. System of any one of the preceding claims, wherein the binning is based on a 2D binning kernel ($\kappa$), wherein a spatial extent of the binning kernel is variable with, and corresponds to, the said change of spatial extent of the projection of the focal spot.

4. System of claim 3, where the spatial extent of the binning kernel is the larger, the larger the spatial extent of the projection of the focal spot.

5. System of any one of the preceding claims, wherein the imaging apparatus is of the radiography type, or of the tomographic type.

6. System of claim 5, wherein the apparatus is any one of: a computed tomography, CT, scanner, a cone beam scanner, CBCT, a C-arm scanner, a radiography apparatus.

7. System of any one of claim 6, wherein the said direction is any one or more of: i) direction of a rotation axis (Z) of the x-ray source and/or of the X-ray detector (XD), and ii) a direction (X) in a plane perpendicular to said, or a rotation axis (Z).

8. System of any one of the preceding claims, wherein at least a part of the system is integrable in the X-ray detector (XD).

9. System of any one of the preceding claims, wherein the system is integrable in detector readout circuitry (RC).

10. System of any one of the preceding claims, wherein the binning is based on calibration data, wherein the calibration data is based on prior recorded X-ray measurements indicative of spatial extent of projection of focal spot.

11. An arrangement (IAR) including any one or more of: a readout-electronics (RC) of the X-ray detector (XD), the X-ray detector (XD), the imaging apparatus (IA), and a memory (MEM') including the calibration data in claim 10.

12. A method of X-ray imaging, comprising:

    receiving (S820) detector data detectable by an X-ray detector (XD) of an X-ray imaging apparatus (IA) upon irradiation by X-radiation from a focal spot (FS) of the imaging apparatus (IA)'s X-ray source (XR); and
    binning (S850) the detector data into binned detector data, and wherein the said binning by binner (BN) is based on a manner in which a spatial extent of a projection on the detector (XD) of the focal spot (FS) is changeable along at least one spatial direction (X,Z).

13. A method of obtaining (S835) calibration data for use in the method of claim 12, wherein the binning is based on such calibration data.

14. A computer program element, which, when being executed by at least one processing unit, is adapted to cause the processing unit to perform the method as per any one of claim 12 or 13.

15. At least one computer readable medium having stored thereon the program element of claim 14.

Fig. 1

Fig. 2A

Fig. 2B

EP 4 721 667 A1

Fig. 3

Fig. 4

EP 4 721 667 A1

Fig. 5

18

Fig. 6

Fig. 7

S810

$\lambda'$

S820

c

S830 ← S835

S840

$\kappa$

S850

S860

$\lambda = b(\lambda')$

S870

S880

Fig. 8

A)

B)

Fig. 9

EP 4 721 667 A1

Fig. 10

Fig. 11

Europäisches
Patentamt

European
Patent Office

Office européen
des brevets

## EUROPEAN SEARCH REPORT

**Application Number**

EP 24 20 4407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/112623 A1 (CARESTREAM HEALTH INC [US]) 29 June 2017 (2017-06-29) * page 9, line 24 - page 29, line 30; figure 10a * ----- | 1-15 | INV. A61B6/00 G06T11/00 ADD. A61B6/03 |
| A | WO 2010/070554 A1 (KONINKL PHILIPS ELECTRONICS NV [NL] ET AL.) 24 June 2010 (2010-06-24) * page 6, line 31 - page 15, line 18 * ----- | 1-15 | |

**TECHNICAL FIELDS SEARCHED (IPC)**

A61B
G06T

The present search report has been drawn up for all claims

| Place of search | Date of completion of the search | Examiner |
|---|---|---|
| Munich | 5 March 2025 | Hooper, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 20 4407

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| WO 2017112623 A1 | 29-06-2017 | EP 3393362 A1 | 31-10-2018 |
| | | US 2018350112 A1 | 06-12-2018 |
| | | WO 2017112623 A1 | 29-06-2017 |
| WO 2010070554 A1 | 24-06-2010 | CN 102256548 A | 23-11-2011 |
| | | EP 2378974 A1 | 26-10-2011 |
| | | JP 5604443 B2 | 08-10-2014 |
| | | JP 2012511988 A | 31-05-2012 |
| | | RU 2011129670 A | 27-01-2013 |
| | | US 2011280367 A1 | 17-11-2011 |
| | | WO 2010070554 A1 | 24-06-2010 |

EPO FORM P0459

For more details about this annex : see Official Journal of the European Patent Office, No. 12/82